# EUROPÄISCHE PATENTSCHRIFT

(11) **EP 2 366 163 B1**
(45) Veröffentlichungstag und Bekanntmachung des Hinweises auf die Patenterteilung: **27.02.2019**
(21) Anmeldenummer: 09796356.5
(22) Anmeldetag: 09.12.2009
(51) Int. Cl.: G06F 19/00, G16H 10/40

(54) **VERFAHREN UND SYSTEM ZUM VERWALTEN VON DATEN VON ANALYSEGERÄTEN**
METHOD AND SYSTEM FOR MANAGING DATA OF ANALYSIS DEVICES
MÉTHODE ET SYSTÈME POUR LA GESTION DE DONNÉES DES DISPOSITIFS D'ANALYSE

(30) Priorität: 12.12.2008 US 121945 P
(43) Veröffentlichungstag der Anmeldung: 21.09.2011
(73) Patentinhaber: Roche Diagnostics GmbH, 68305 Mannheim (DE); F. Hoffmann-La Roche AG, 4070 Basel (CH)
(72) Erfinder: JANSCHITZ, Wolfgang, A-8045 Graz (AT); GROMANN, Klaus, A-8046 Graz (AT); KARGL, Christian, A-8020 Graz (AT)
(74) Vertreter: Margotti, Herwig Franz
(86) Internationale Anmeldenummer: PCT/EP2009/066774
(87) Internationale Veröffentlichungsnummer: WO 2010/066816

(56) Entgegenhaltungen:
- US-A1- 2001 051 952
- US-A1- 2006 041 450
- US-A1- 2006 271 607
- US-A1- 2007 265 884
- US-A1- 2007 271 316
- US-A1- 2008 262 776
- US-B1- 6 961 617

## Beschreibung

Die Erfindung betrifft ein Verfahren zum Verwalten von Daten von Analysegeräten, insbesondere zum Transfer von Daten zwischen einem erstem und zweiten Analysegerät.

Die Erfindung betrifft weiterhin ein Analysegerät und ein System mit Analysegeräten.

Des Weiteren betrifft die Erfindung ein Computerprogrammprodukt mit dessen Hilfe das eingangs erwähnte Verfahren auf einem programmierbaren Analysegerät durchführbar ist.

Ein bevorzugtes Anwendungsgebiet solcher Analysegeräte oder Analysatoren liegt auf dem medizinisch-diagnostischen Gebiet, wo solche Geräte beispielsweise zur Analyse von Körperflüssigkeiten, insbesondere Blutanalyse, eingesetzt werden.

Häufig werden solche Analysatoren zur dezentralen Bestimmung von Point of Care (POC)-Parametern, beispielsweise der Blutgase (O₂, CO₂, pH), der Elektrolyte (K⁺, Na⁺, Ca⁺⁺, Cl⁻) der Metabolite (Glukose und Laktat), des Hämatokrits, der Hämoglobinparameter (tHb, SO₂, etc.) und Bilirubin, eingesetzt. Als Probenmaterial dient hierbei zumeist humanes Vollblut, aber auch Anwendungen in der Veterinärmedizin und die Verwendung von Serum-, Plasma-, Harn- und Dialysatproben sind möglich.

Solche Analysatoren weisen entsprechende Messelemente und damit verbundene Recheneinheiten, wie beispielsweise programmierbare Mikrocomputer oder auch applikationsspezifische Schaltkreise auf, welche die Funktionen der Analysatoren steuern und demgemäß eine Steuerstufe bilden. Die Analysatoren weisen weiters Speichereinheiten (z.B. eine 2,5" Festplatte als primärer Datenspeicher) auf, die auch als internes Speichermedium bezeichnet werden. Mit ihrer Hilfe werden üblicherweise alle (auch vom Benutzer veränderbare) Einstellungen (Konfigurationsdaten) sowie Messergebnisse (Messdaten), Benutzerdaten und Patienten- bzw. Probandendaten intern gespeichert. Manche dieser Daten können eventuell in andere Systeme oder auf andere Datenspeicher auf Anforderung exportiert bzw. aus anderen Systemen oder von anderen Datenspeichern importiert werden. Für den Datenaustausch mit Labor- und Krankenhausinformationssystemen oder diversen Server-/Client-basierten Daten- und Geräteverwaltungssystemen existieren Standardprotokolle wie ASTM E 1394, 91 oder POCT1-A oder HL7.

Auch in der deutschen Gebrauchsmusterschrift DE 201 13 153 U1 wird auf die Problematik des Austausches von Daten bei einem elektrochemischen Analysegerät eingegangen. Um den Austausch von Daten auf einfache Weise zu ermöglichen, wird dort eine Daten-Lese- und -Schreibeinheit mit einer Wechsel-Halbleiterspeicher-Karte als Lösung offenbart. Diese Daten-Lese- und -Schreibeinheit und die Karte ersetzen im bekannten Fall eine Schnittstelle zur Übertragung von Messdaten und Geräteparametern über ein entsprechendes Kommunikationsnetz zu einer zentralen Datenverarbeitungsstation.

An dieser Stelle sei weiters erwähnt, dass in der US 2006/0294420 A1 ein Verfahren zum Datensichern und Wiederherstellen der Daten im Zusammenhang mit einem PC offenbart ist. Eine solche Datensicherung erfolgt üblicherweise zeitgesteuert, oder sie wird manuell ausgelöst. Bei diesem Verfahren werden Konfigurationsdaten, die zum Wiederherstellen der gesicherten Daten benötigt werden, isoliert von den zu sichernden Daten behandelt, um diese Konfigurationsdaten im Fall des Wiederherstellens der Daten leichter wieder aufzufinden.

Des Weiteren sei an dieser Stelle noch auf die US 2006/0148463 A1 hingewiesen, in der auf das Versagen eines Mobiltelefons eingegangen wird, wobei das Versagen das Mobiltelefons hauptsächlich durch falsche oder fehlerhafte Einstellungen verursacht wurde. In diesem Kontext stellt sich die Aufgabe des Wiederherstellens von originalen Einstellungen. Diese Aufgabe wird durch ein Monitoring-Modul gelöst, das eine Vielzahl von ursprünglichen Einstellungen speichert, sobald eine SIM-Karte in ein Mobiltelefon eingesetzt wird, und die Originaleinstellungen wieder herstellt, wenn die Einstellungen des Mobiltelefons nicht mit den Originaleinstellungen übereinstimmen.

Die US 2007/271316 A1 offenbart ein Verfahren und eine Einrichtung zur Speicherung und Sicherung von Patientendaten (z.B. Röntgenbildern) auf Archiv-Speichermedien, die untereinander vernetzt und geografisch voneinander getrennt sind. Die in unterschiedlichen Verarbeitungseinrichtungen (z.B. Bildspeicherdatenbank, Spitalsinformationssytem, Eingabecomputer des Arztes) gespeicherten Patientendaten werden über ein Netzwerk-Interface-Device an das eine bzw. die mehreren Archiv-Speichermedien übermittelt und in diesen gespeichert. Entsprechend vorgegebenen Back-up Rules werden neue oder veränderte Patientendaten in den Archiv-Speichermedien gespeichert.

Weder die oben angesprochenen Protokolle noch die vorstehend erörterten Offenbarungen in der Patent- und Gebrauchsmusterliteratur lösen die inhärenten Probleme des Datentransfers bei bekannten Analysatoren. Diese bisher eingesetzten Datentransferverfahren weisen insbesondere folgende Nachteile auf:
- manuelle Eingriffe sind notwendig (z. B. Auswahl der Daten, Starten der Backup-Applikation).
- ein manuelles Backup-Verfahren impliziert im Bedarfsfall in der Regel Datenverlust, da der Datenexport nicht unmittelbar vor Eintreten eines Gerätedefekts durchgeführt wird.
- eventuell ist eine eigene Ver- und Entschlüsselung der Backup-Daten notwendig.
- in Hardware realisierte Lösungen (Druckercontroller, Disc-Controller, teilweise eigene Prozessoren und Interfaces zu Speichermedien) sind in der Regel teuer.

Es ist daher eine Aufgabe der Erfindung ein Verfahren, ein Analysegerät, ein System und ein Computerprogrammprodukt bereitzustellen, mit welchen es insbesondere im Falle eines Schadens an einem Analysegerät, wie beispielsweise an der Steuerstufe (Recheneinheit) oder am internen Speichermedium (Datenspeicher), möglich ist, alle relevanten Daten auf ein Ersatz-Analysegerät zu transferieren, um damit den Analysebetrieb so rasch und unproblematisch wie möglich fortzusetzen.

Diese Aufgabe wird durch ein Verfahren gemäß Patentanspruch 1, durch ein Analysegerät gemäß Patentanspruch 9, durch ein Computerprogrammprodukt gemäß Patentanspruch 11 sowie durch ein System gemäß Patentanspruch 12 gelöst.

Bei einem erfindungsgemäßen Verfahren zum Verwalten von Daten von Analysegeräten, insbesondere zum Transfer von Daten zwischen einem ersten und zweiten Analysegerät, ist vorgesehen, dass das erste Analysegerät Daten in einem internen Speichermedium neu anlegt oder modifiziert und während des Betriebs fortlaufend die neu angelegten oder modifizierten Daten in einem nichtflüchtigen Wechsel-Speichermedium redundant speichert, und dass die auf dem Wechsel-Speichermedium von einem ersten Analysegerät redundant gespeicherten Daten in das interne Speichermedium eines zweiten Analysegeräts importiert werden.

Ein erfindungsgemäßes Analysegerät, das Daten verwaltet und zum Durchführen des erfindungsgemäßen Verfahrens ausgebildet ist, besitzt ein internes Speichermedium zum Speichern der Daten, die in dem Analysegerät neu angelegt oder modifiziert werden, und ein Speicherinterface zum Zugreifen auf ein nichtflüchtiges Wechsel-Speichermedium, sodass mit Hilfe des Speicherinterfaces während des Betriebs des Analysegeräts fortlaufend die neu angelegten oder modifizierten Daten in dem Wechsel-Speichermedium redundant speicherbar sind. Das Analysegerät ist ein medizinisch-diagnostisches Analysegerät, wie beispielsweise ein Analysegerät zur Analyse von Körperflüssigkeiten, insbesondere ein Blutanalysegerät.

Unter dem Begriff "interner Speicher", wie hierin verwendet, ist ein flüchtiger oder nichtflüchtiger Speicher zu verstehen, der während des Betriebs des Analysegeräts nicht entfernt werden darf und zum Betrieb des Analysegeräts benötigt wird. Der interne Speicher kann z.B. als Halbleiterspeicher oder magnetischer Speicher (Festplatte) oder optischer Speicher ausgeführt sein.

Das Wechsel-Speichermedium ist ein nichtflüchtiges Speichermedium, das nicht für die Grundfunktionalität des Analysegeräts, z.B. zur Durchführung von Analysen benötigt wird,
sondern ausschließlich auf dem internen Speicher des ersten Analysegeräts vorhandene Daten redundant speichert. Das erste Analysegerät verwendet die auf dem Wechsel-Speichermedium redundant gespeicherten Daten nicht zu seinem Betrieb. In diesem Sinn ist das Wechsel-Speichermedium unabhängig vom internen Speicher. Wie weiter unten ausgeführt ist, kann allerdings aus Sicherheitsgründen vorgesehen werden, dass das Analysegerät nur dann den Betrieb aufnimmt oder fortsetzt, wenn der Zugriff auf das Wechsel-Speichermedium möglich ist.

Des Weiteren ist bei einem erfindungsgemäßen Computerprogrammprodukt vorgesehen, dass das Computerprogrammprodukt direkt in einen Arbeitsspeicher eines programmierbaren Analysegeräts ladbar ist und Programmcodeabschnitte aufweist, die, wenn das Computerprogrammprodukt mit Hilfe des Analysegeräts ausgeführt wird, das Verfahren gemäß der Erfindung realisieren.

Durch das Vorsehen der erfindungsgemäßen Maßnahmen ist auf vorteilhafte Weise erreicht, dass die relevanten Daten im Betrieb des Analysegeräts fortlaufend zusätzlich zum Speichern auf ein primäres internes Speichermedium auch redundant auf einem zweiten, nicht-flüchtigen Wechsel-Speichermedium gespeichert werden, wobei das zweite Speichermedium nicht in das Analysegerät integriert ist. Da das interne Speichermedium und das Wechsel-Speichermedium unabhängig voneinander sind, also keine funktionelle Verknüpfung zwischen ihnen besteht, können Datenfehler, die zufällig auf dem internen Speichermedium beim Anlegen und/oder Modifizieren der Daten entstehen, nicht auf das Wechsel-Speichermedium propagieren. Gemäß der Erfindung ist vorgesehen, dass das Analysegerät während seines Betriebs fortlaufend die neu angelegten und/oder modifizierten Daten in einem nichtflüchtigen Wechsel-Speichermedium redundant speichert. Darunter wird verstanden, dass die Daten unmittelbar nach deren Entstehung (z.B. im internen Speichermedium oder sogar direkt in der Steuerstufe) redundant auf das zusätzliche Wechsel-Speichermedium geschrieben werden. Dabei wird keine Datenspiegelung durchgeführt, sondern die Daten werden unabhängig von dem anderen Speichermedium geschrieben. Dies macht auch den Unterschied zu einem "Redundant Array of Independent Disks" (RAID) System aus. Ein solches RAID-System dient nämlich zur Organisation von mehreren physikalischen Festplatten eines Computers zu einem logischen Laufwerk, das eine höhere Datensicherheit bei Ausfall einzelner Festplatten gewährt. So werden beispielsweise bei einem Verbund von zwei Festplatten, die das logische Laufwerk bilden, auf alle Festplatten die gleichen Daten gespiegelt, also redundant angelegt. Eine Spiegelplatte ist jedoch kein Ersatz für eine Datensicherung im herkömmlichen Sinne, da sich auch versehentliche oder fehlerhafte Schreiboperationen augenblicklich auf die Spiegelplatten übertragen. Im Gegensatz dazu werden also gemäß der Erfindung Daten unabhängig voneinander auf voneinander unabhängige Speichermedien geschrieben, so dass die dem RAID-System inhärenten Probleme vermieden sind.

In einer bevorzugten Ausführungsform ist das nicht-flüchtige zweite (externe) Speichermedium, also das Wechsel-Speichermedium, eine CompactFlash-Karte. Prinzipiell können jedoch alle möglichen externen, nicht-flüchtigen Speichermedien erfindungsgemäß eingesetzt werden, u.a. beispielsweise andere Speicherkarten, USB-Sticks oder auch externe Festplatten. Diese können direkt in entsprechende Einschübe des Analysegeräts eingesetzt oder mittels entsprechender Anschlüsse/Schnittstellen mit dem Analysegerät verbunden werden. Es können auch Ausführungsformen verwirklicht werden, in welchen die nichtflüchtigen zweiten (externen) Speichermedien über ein Netzwerk mit dem Analysegerät oder den Analysegeräten verbunden sind, wie beispielsweise dann, wenn die externen, nichtflüchtigen Speichermedien als Netzwerkspeicher oder Netzwerkfestplatten ausgebildet sind. Falls sowohl das erste als auch ein zweites Analysegerät über ein Netzwerk mit dem externen, nicht-flüchtigen Wechsel-Speichermedium verbunden sind, ist in diesem Falle kein physikalischer Transfer (Wechsel) des externen Wechsel-Speichermediums zwischen den Analysegeräten notwendig. Vielmehr kann der Datentransfer hier durch eine entsprechende Zugriffsberechtigung des äquivalenten zweiten Analysegeräts auf die entsprechenden relevanten redundanten Daten des ersten Analysegeräts erfolgen. Daher wurde auch die Bezeichnung "Wechsel-Speichermedium" gewählt, um anzuzeigen, dass im Gegensatz zu dem internen Speichermedium das nicht integrierte (zweite) Speichermedium in Bezug auf die beiden Analysegeräte wechselweise verwendet werden kann.

In einer bevorzugten Ausführungsform der Erfindung werden zur Durchführung des erfindungsgemäßen Verfahrens Analysegeräte eingesetzt, welche entsprechende Einschübe (beispielsweise einen CompactFlash-Karten-Schacht) oder Schnittstellen zum Verbinden externer nicht-flüchtiger Speichermedien aufweisen und weiterhin entsprechende Steuerelemente (Speicherelemente, Schreibelemente, Leseelemente, Datenverarbeitungselemente...) aufweisen.

Mit Hilfe der Erfindung ist daher auf vorteilhafte Weise erreicht, dass im Fall einer Störung oder eines Schadensfalls an dem ersten Analysegerät die Daten rasch und unproblematisch auf das zweite Analysegerät transferiert werden können. Für den benötigten Datentransfer von dem ersten Analysegerät auf das zweite Analysegerät muss nur z.B. die Flash-Karte aus dem ersten Analysegerät entnommen und in das zweite Analysegerät eingesetzt werden. Die auf der Flash-Karte gespeicherten Daten werden in den internen Speicher des zweiten Analysegeräts kopiert. Das zweite Analysegerät übernimmt dadurch sämtliche gespeicherte Daten des ersten Analysegeräts, die unmittelbar vor dem Störfall vorlagen. In Folge übernimmt die in dem zweiten Analysegerät eingesetzte Flash-Karte die Funktion des redundanten Wechsel-Speichermediums. Die z.B. mit dem ersten Analysegerät durchgeführten Analysen können daher faktisch ohne längere Unterbrechungen und praktisch übergangslos mit dem zweiten Analysegerät fortgesetzt werden, wobei zugleich die Datenkonsistenz erhalten bleibt.

Weitere, besonders vorteilhafte Ausgestaltungen und Weiterbildungen der Erfindung ergeben sich aus den abhängigen Ansprüchen sowie der nachfolgenden Beschreibung.

Der Anwendungsbereich des Verfahrens liegt insbesondere im Bereich der medizinisch-diagnostischen Analysegeräte, wie dies beispielsweise bei Analysegeräten zur Analyse von Körperflüssigkeiten, insbesondere Blutanalysegeräten der Fall ist. In einer bevorzugten Ausführungsform sind bei einem erfindungsgemäßen System ein erstes Analysegerät und das zu ihm äquivalente zweite Analysegerät durch baugleiche Analysatoren gebildet.

Um sicherzugehen, dass für den Betrieb des äquivalenten Analysegeräts alle nötigen Daten vorhanden sind, hat es sich als vorteilhaft erwiesen, dass - je nach Situation und Anwendung - die Daten zumindest eine der nachfolgend angeführten Datenart oder eine Mehrzahl der nachfolgend angeführten Datenarten repräsentieren, nämlich:
i) Konfigurationsdaten,
ii) Messdaten,
iii) Probandendaten,
iv) Probendaten, und
v) Benutzerdaten.

Unter Konfigurationsdaten sind insbesondere Geräteeinstellungen, wie Sprache, IP-Adresse, Anzeigebildschirm, Ländereinstellungen, Energiemanagement, Netzwerkeinstellungen, berechnete und angezeigte Maßeinheiten, verwendete Messparameter, Mess-, Kalibrations- und Qualitätskontrollgrenzen, Qualitätsmessungs-Materialien, Sicherheitseinstellungen, Audioeinstellungen, geplante automatische Durchführung von Analysatorfunktionen, Peripheriegeräte, etc. zu verstehen.

Messdaten umfassen Messresultate, Rohdaten, Bilddaten, Messsignale, weiterverarbeitete Daten und dergleichen.

Probandendaten umfassen insbesondere Patienten-Identifikationskennungen.

Probendaten umfassen insbesondere Proben-Identifikationskennungen, Probennahmezeitpunkt, Probentyp und/oder Abnahmetemperatur.

Benutzerdaten umfassen insbesondere Benutzer-Identifikationskennungen, benutzerspezifische Berechtigungen, Benutzerprofile und dergleichen.

Da in einer bevorzugten Ausführungsform auch praktisch alle Datenarten redundant mitgeschrieben werden können, sind keine regelmäßigen Synchronisationszyklen zwischen den Speichermedien nötig. Für Kalibrations- und Qualitätskontrolldaten, die rein gerätespezifisch sind (und somit auf dem äquivalenten Analysegerät nicht ohne weiteres eingesetzt werden sollten), bestehen folgende Möglichkeiten:
a) Kalibrations- und Qualitätskontrolldaten, die rein gerätespezifisch sind, werden nicht auf das Wechsel-Speichermedium (z.B. Compact-Flash Karte) geschrieben.
b) Kalibrations- und Qualitätskontrolldaten, die rein gerätespezifisch sind, werden auch auf das Wechsel-Speichermedium geschrieben (und es wird über ihre Verwendung erst beim in Betrieb nehmen des äquivalenten Analysegeräts entschieden).

Die bei der medizinischen oder anders gearteten Datengewinnung typischerweise geforderte Datensicherheit bzw. Vertraulichkeit der Daten kann auf vorteilhafte Weise dadurch erhalten werden, wenn zumindest die in dem Wechsel-Speichermedium gespeicherten Daten verschlüsselt werden. Weiters können die Daten durch Passwörter und/oder Benutzernamen gegen Missbrauch gesichert sind. Dadurch sind die redundanten Daten optional vor unbefugtem Zugriff geschützt.

In einer Ausführungsform des erfindungsgemäßen Verfahrens exportiert das erste Analysegerät bei Verbindung mit einem neuen Wechsel-Speichermedium die in dem internen Speichermedium gespeicherten Daten dann in das neue Wechsel-Speichermedium, wenn dieses nicht bereits valide Daten von einem Fremd-Analysegerät enthält. Man verhindert dadurch unabsichtliches Überschreiben von gültigen Daten auf dem Wechsel-Speichermedium.

Um eine Bindung zwischen dem Analysegerät und dem Wechsel-Speichermedium zu erhalten, was auch als Tupel bezeichnet wird, kann vor der ersten Inbetriebnahme (im gerätespezifischen Normalbetrieb, wie z.B. Mess- und/oder Analysebetrieb) des Analysegeräts eine eindeutige Identifikationskennung (z.B. Seriennummer) des Wechsel-Speichermediums, das ihm z.B. bei der Fertigung oder Auslieferung zugeordnet wird, in Konfigurationsdaten des Analysegeräts abgespeichert werden. Alternativ kann vor der ersten Inbetriebnahme des Analysegeräts eine eindeutige Identifikationskennung (z.B. Seriennummer) des Analysegeräts, das ihm z.B. bei der Fertigung oder Auslieferung zugeordnet wird, auf dem Wechsel-Speichermedium abgespeichert werden, um eine Zuordnung der auf dem Wechsel-Speichermedium gespeicherten Daten zu deren Ursprung, d.h. von welchem Analysegerät diese stammen, herzustellen.

Optional kann aus Sicherheitsgründen die Existenz eines eindeutig identifizierten externen (Wechsel)-Speichermediums in dem Analysegerät zum Zeitpunkt des Systemstarts und dessen Überprüfung als Sicherungsmaßnahme erforderlich sein, um das Analysegerät in Betrieb gehen zu lassen. Wird das externe Speichermedium im laufenden Betrieb aus dem Analysegerät entfernt, so kann dies durch einen Fehler beim nächsten Schreibversuch erkannt werden. Das Analysegerät kann daraufhin in einen Systemstop-Zustand gehen und erst wieder bedient werden, wenn das korrekte (Wechsel)-Speichermedium in ihm vorhanden ist. Daher ist es von Vorteil, wenn insbesondere bei einer ersten Inbetriebnahme des Analysegeräts die Verfügbarkeit des Wechsel-Speichermediums geprüft wird und, wenn das Wechsel-Speichermedium nicht verfügbar ist, der Betrieb nicht oder nur eingeschränkt, insbesondere jedoch ohne redundantes Speichern der relevanten Daten auf dem Wechsel-Speichermedium zugelassen wird.

Um zu gewährleisten, dass ein Analysegerät nicht mit jedem beliebigen Wechsel-Speichermedium zum Zweck der redundanten Speicherung von Daten in Betrieb genommen werden kann, hat es sich als zuverlässige Maßnahme erweisen, dass das Wechsel-Speichermedium einen Hardware-Token umfasst, der für den Betrieb des Analysegeräts nötig ist.

Wenn festgestellt wird, dass das Wechsel-Speichermedium verfügbar ist, wird geprüft, ob ein auf dem Wechsel-Speichermedium vorhandener Datenbestand aus einem anderen Analysegerät stammt und, wenn dies zutrifft, werden diese in dem Wechsel-Speichermedium enthaltenen redundant gespeicherten Daten in das interne Speichermedium des Analysegeräts importiert.

Die Entscheidung, ob in dem Wechselspeichermedium redundant gespeicherte Daten in das Analysegerät importiert werden, kann aus Sicherheitsgründen auch von einer Benutzerinteraktion abhängig gemacht werden. Für den Fall, dass festgestellt wird, dass das Wechsel-Speichermedium leer ist, kann die Benutzerinteraktion durch eine automatische Entscheidung, nämlich Daten von dem Analysegerät auf das Wechsel-Speichermedium zu exportieren, ersetzt werden. Betreffend den Importvorgang ist es zur Nachvollziehbarkeit des Datenimports von Vorteil, wenn diese Vorgänge protokolliert werden, was vorzugsweise mit Hilfe eines sogenannten Audit-Trail erfolgen kann.

Die Durchführung des erfindungsgemäßen Verfahrens erfolgt gemäß einem bevorzugten Ausführungsbeispiel mit Hilfe eines erfindungsgemäßen Analysegeräts, das programmierbar ist. Dieses Analysegerät weist üblicherweise einen Programmspeicher, einen Arbeitsspeicher und einen Mikroprozessor auf, wobei der Mikroprozessor eine Steuerstufe des Analysegeräts bildet und mit dem internen Speicher und dem Speicherinterface gekoppelt ist. Mit Hilfe des Programmspeichers wird ein Computerprogramm gespeichert, das, wenn es mit Hilfe des Mikroprozessors ausgeführt wird, das Verfahren gemäß der Erfindung realisiert. Die Durchführung des Verfahrens kann jedoch auch mit Hilfe eines Analysegeräts erfolgen, bei dem ein anwendungsspezifischer (z.B. fix verdrahteter) Schaltkreis zur Anwendung kommt, dessen Logik das erfindungsgemäße Verfahren repräsentiert.

Weiters ist ein System mit zwei erfindungsgemäßen Analysegeräten vorgesehen, wobei die in einem der beiden Analysegeräte während des Betriebs des einen Analysegeräts fortlaufend neu angelegten und/oder modifizierten Daten und in dem Wechsel-Speichermedium redundant gespeicherten Daten zum Betrieb des anderen Analysegeräts dienen, wenn das andere Analysegerät auf das Wechsel-Speichermedium zugreift.

Die Erfindung wird im Folgenden unter Hinweis auf die beigefügten Figuren anhand eines Ausführungsbeispiels, auf welches die Erfindung jedoch nicht beschränkt ist, noch einmal näher erläutert. Dabei sind in den verschiedenen Figuren gleiche Komponenten mit identischen Bezugsziffern versehen.

Es zeigen:
- Figur 1: stark schematisiert und in Form eines Blockschaltbildes ein Analysegerät gemäß einem Ausführungsbeispiel der Erfindung,
- Figur 2: in Form eines Ablaufdiagramms ein Verfahren gemäß der Erfindung,
- Figur 3: stark schematisiert das Analysegerät in einer ersten Perspektive,
- Figur 4: das Analysegerät gemäß der Figur 3 in einer zweiten Perspektive und
- Figur 5: ein Detail des Analysegeräts gemäß der Figur 3.

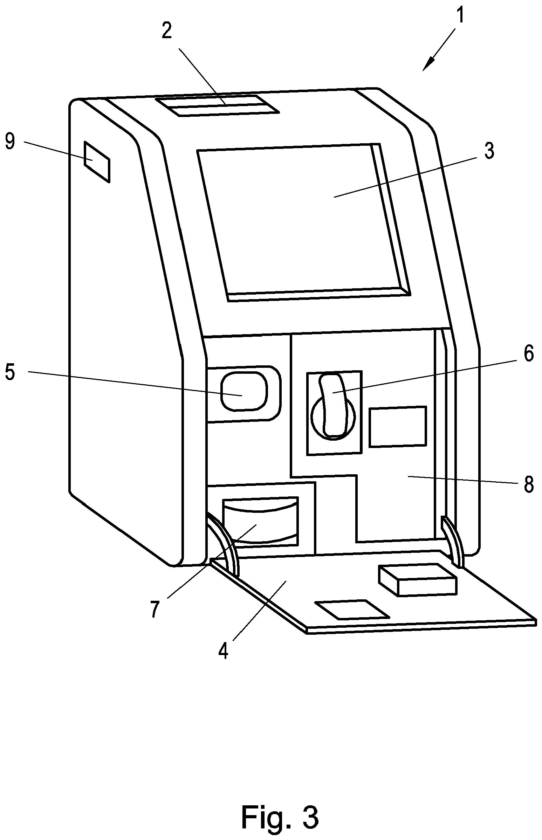

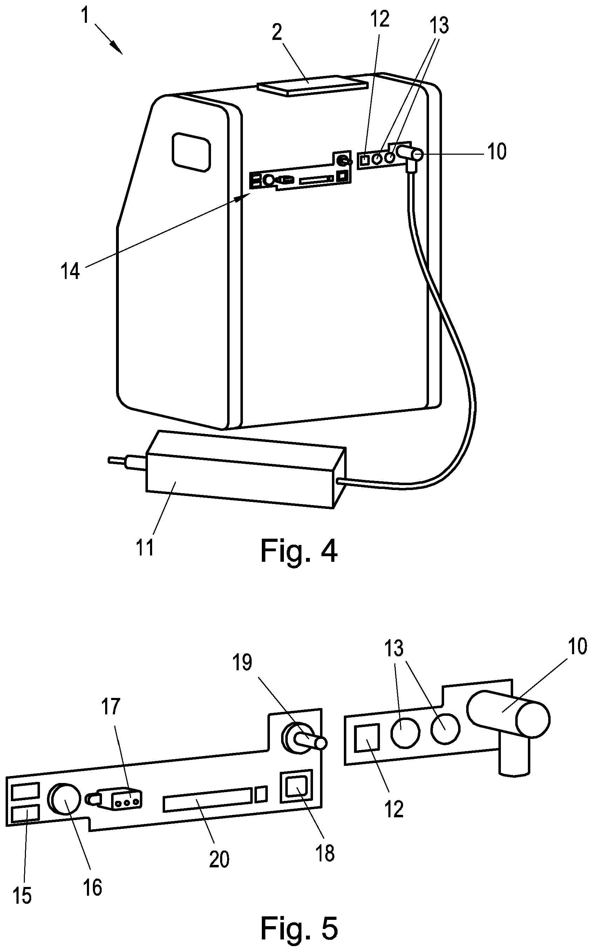

Figuren 3 und 4 zeigen einen möglichen Aufbau und die von außen sichtbaren Bestandteile eines medizinischen Blutgasanalysators, der das Analysegerät 1 realisiert. In der Figur 3 ist das Analysegerät 1 im Wesentlichen von vorne dargestellt. Auf der Oberseite des Analysegeräts 1 befindet sich ein Drucker 2, der einen Teil eines Benutzerinterfaces bildet. Ebenfalls Teil dieses Benutzerinterfaces ist ein Bildschirm 3, bevorzugt ein Touchscreen-Bildschirm, der im Wesentlichen an der Vorderseite des Analysegeräts 1 angeordnet ist. An besagter Vorderseite befindet sich auch eine Gerätetür 4, die im geöffneten Zustand dargestellt ist. Die geöffnete Gerätetür 4 gibt den Blick auf ein Messmodul 5 mit einer Sensor Cartridge, ein Probeneingabemodul 6, ein AutoQC-Modul 7 und ein Fluid Pack 8 frei. An der rechten Seite des Analysegeräts 1 befindet sich eine USB-Schnittstelle 9. In der Figur 4 ist das Analysegerät 1 im Wesentlichen von hinten dargestellt. Auf der Hinterseite befindet sich ein Netzanschluss 10, an den ein Netzgerät 11 angeschlossen ist. Mit Hilfe eines Netzschalters 12 lässt sich das Analysegerät Vorrichtung ein- bzw. ausschalten. Zwischen dem Netzschalter 12 und dem Netzanschluss 10 befinden sich zusammengefasst unter dem Bezugszeichen 13 zwei Sicherungen und zwei Status-LEDs. Des Weiteren ist ein Schnittstellenbereich 14 dargestellt, auf den in der Figur 5 im Detail eingegangen ist.

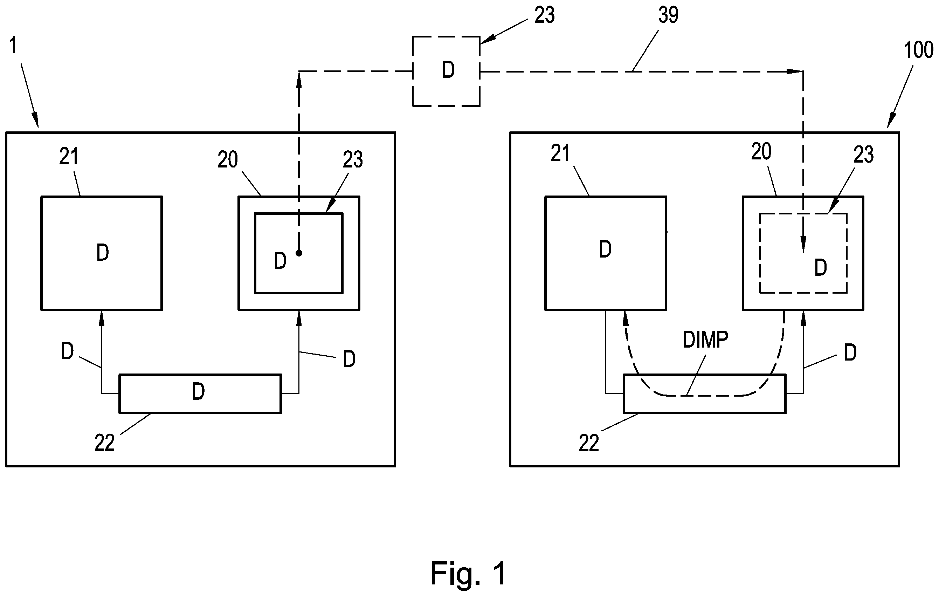

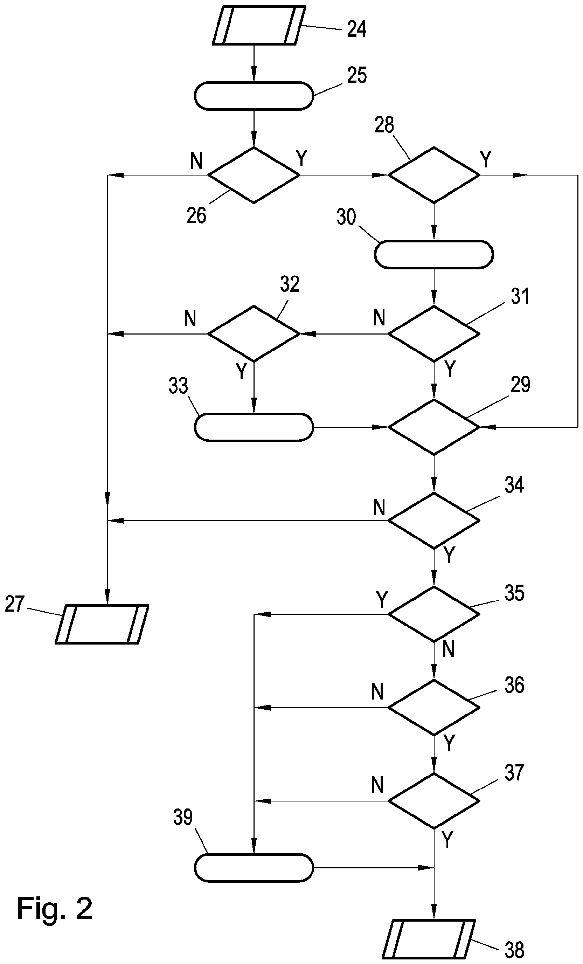

Der in der Figur 5 dargestellte Schnittstellenbereich weist zwei USB-Anschlüsse 15, einen Anschluss für einen Barcode Scanner 16, einen RS 232 Servicestecker 17, einen RJ 45 Netzwerkanschluss 18, einen Potentialabgleichstecker 19 und ein Speicherinterface 20 für eine Flash-Karte auf. Auf die Funktion des Speicherinterfaces 20 wird nachfolgend noch im Detail anhand der Figur 1 und der Figur 2 eingegangen.

In der Figur 1 ist ein Blockschaltbild des Analysegeräts 1 und eines dazu äquivalenten Analysegeräts 100 mit den für die Erörterung der gegenständlichen Erfindung nötigen strukturellen Elementen dargestellt. Im vorliegenden Fall ist das äquivalente Analysegerät 100 baugleich zu dem Analysegerät 1 ausgeführt, was jedoch nicht zwingend nötig ist. Das Analysegerät 1 weist neben dem Speicherinterface 20 ein internes Speichermedium 21 und eine Steuerstufe 22 auf. Auf die dem Fachmann hinlänglich bekannte Kopplung des Speicherinterfaces 20 und des internen Speichermediums 21 mit der Steuerstufe 22 wird an dieser Stelle nicht weiter eingegangen. Im Unterschied zu dem äquivalenten Analysegerät 100 ist im Analysegerät 1 eine Flash-Card 23 eingelegt, sodass die in dem internen Speichermedium 21 neu angelegten und/oder modifizierten Daten D auch in der Flash-Karte 23 redundant mitprotokolliert werden können. Mit Hilfe der Analysegeräte 1 bzw. 100 lässt sich nun das erfindungsgemäße Verfahren durchführen, so wie dies mit Hilfe der Figur 2 nachfolgend erörtert ist.

Der Beginn des in der Figur 2 dargestellten Verfahrens ist durch einen Block 24 gekennzeichnet. Bei einem darauf folgenden Block 25 wird die Konfiguration des Analysegeräts 1 gelesen. Danach wird zu einem Block 26 verzweigt, bei dem geprüft wird, ob eine Flash-Karte 23 in dem Speicherinterface 20 verfügbar ist. Falls dies nicht der Fall ist, wird der Betrieb des Analysegeräts 1 im Block 27 mit einer Fehlermeldung gestoppt. Alternativ dazu könnte ein eingeschränkter Betrieb des Analysegeräts 1 zugelassen werden, bis schließlich eine Flash-Karte 23 eingelegt wird.

Im Fall des Analysegeräts 1 ist das Ergebnis dieser Prüfung positiv und das Verfahren wird bei einem Block 28 fortgesetzt, bei dem geprüft wird, ob die Flash-Karte 23 leer ist. Wenn die Flash-Karte 23 leer ist, so wird die weitere Abarbeitung der Startprozedur in einem Block 29 fortgesetzt. Wenn die Flash-Karte 23 nicht leer ist, so wird in einem Block 30 die Konfiguration der Flash-Karte 23 eingelesen und anschließend in einem Block 31 überprüft, ob die Seriennummer der eingelegten Flash-Karte 23, die in der Konfiguration der Flash-Karte 23 enthalten ist, dieselbe geblieben ist wie beim letzten Start-up des Analysegeräts 1. Alternativ kann hier überprüft werden, ob die Seriennummer oder eine andere Identifikationskennung des Analysegeräts 1 mit einer auf der Flash-Karte 23 gespeicherten Seriennummer oder Identifikationskennung eines Analysegeräts übereinstimmt.

Wenn die Seriennummer der eingelegten Flash-Karte 23 mit der im internen Speicher des Analysegeräts hinterlegten Seriennummer einer Flash-Karte 23 (oder alternativ die auf der Flash-Karte 23 gespeicherte Seriennummer des Analysegeräts 1 mit der Seriennummer des momentan verwendeten Analysegeräts) identisch ist, was bedeutet, dass die Flash-Karte 23 sich weiterhin im bisher verwendeten Analysegerät 1 befindet, so wird die Startprozedur in dem Block 29 fortgesetzt. Falls sich die Seriennummer der eingelegten Flash-Karte 23 (oder alternativ die auf der Flash-Karte 23 gespeicherte Seriennummer des Analysegeräts 1) und damit auch die bisherige Zuordnung von Flash-Karte 23 und Analysegerät 1 geändert hat, so wird zu einem Block 32 verzweigt, der eine Benutzerinteraktion darstellt, in der der Benutzer einem Import der auf der Flash-Karte 23 gespeicherten Daten in das interne Speichermedium des Analysegeräts 1 zustimmen oder dies ablehnen kann. Lehnt der Benutzer dies ab, so wird der Betrieb des Analysegeräts 1 im Block 27 mit einer Fehlermeldung gestoppt. Stimmt der Benutzer dem Import der Daten zu, so erfolgt im Block 33 der Datenimport in das interne Speichermedium des Analysegeräts 1, wobei eine aktuelle Zuordnung von Wechsel-Speichermedium und Analysegerät über entsprechende Hinterlegung von Identifikationskennungen von Wechsel-Speichermedium oder Analysegerät erfolgt und die Startprozedur wird in dem Block 29 fortgesetzt. Die Benutzerinteraktion 32 stellt ein zusätzliches Sicherheitsfeature dar, dass optional auch weggelassen werden kann.

Der Block 29 markiert den Übergang von einer Basis-Initialisierung des Analysegeräts 1 der eigentlichen Start-up Prozedur zu einem Beginn des Daten-Logging, bei dem in einem Block 34 nochmals das Vorhandensein der Flash-Karte 23 in dem Analysegerät 1 abgefragt wird und bei nicht vorhandener Flash-Karte 23 der Betrieb des Analysegeräts 1 im Block 27 mit einer Fehlermeldung gestoppt wird.

Bei vorhandener Flash-Karte 23 wird in einem Block 35 nochmals geprüft, ob die Flash-Karte 23 leer ist. Wenn die Flash-Karte 23 leer ist, so werden im Block 39 die in dem internen Speichermedium des Analysegeräts 1 gespeicherten, neu abgelegten und/oder modifizierten Daten D in die Flash-Karte 23 exportiert und anschließend im Block 38 das weitere Start-up des Analysegeräts fortgesetzt, das für die Zwecke der vorliegenden Erfindung aber nicht mehr von Bedeutung ist.

Ist die Flash-Karte 23 nicht leer, so wird in einem Block 36 geprüft, ob die Software-Version der Flash-Karte 23 mit jener des Analysegeräts 1 übereinstimmt, d.h. ob die auf der Flash-Karte 23 vorliegenden Daten in der gleichen Struktur vorliegen, wie sie auch im Analysegerät 1 vorliegen. Falls dies nicht zutrifft, so wird zum Block 39 verzweigt, in dem die in dem internen Speichermedium des Analysegeräts 1 gespeicherten Daten in die Flash-Karte 23 exportiert werden; anschließend wird im Block 38 das weitere Start-up des Analysegeräts fortgesetzt.

Falls die Software-Version der Flash-Karte 23 mit jener des Analysegeräts 1 übereinstimmt, so wird in einem Block 37 überprüft, ob die Datenbasis der Flash-Karte 23 mit jener des internen Speichermediums 21 des Analysegeräts 1 übereinstimmt, d.h. ob die auf der Flash-Karte 23 vorliegenden Daten mit denen im Analysegerät 1 vorliegenden Daten übereinstimmen.. Falls die Datenbasen nicht übereinstimmen, so wird zum Block 39 verzweigt, in dem die in dem internen Speichermedium des Analysegeräts 1 gespeicherten Daten in die Flash-Karte 23 exportiert werden; anschließend wird im Block 38 das weitere Start-up des Analysegeräts fortgesetzt. Falls die Datenbasen übereinstimmen, so wird sofort zum Block 38 verzweigt.

Nach Abschluss des Start-up geht das Analysegerät 1 in einen Betriebsmodus über, in dem fortlaufend neu angelegte und/oder modifizierte Daten D auf der Flash-Karte 23 redundant gespeichert werden. Es werden also alle im Fehlerfall zu transferierenden Daten D (z.B. Konfigurationsdaten, Benutzerdaten, Patientendaten und Messergebnisse) im laufenden Betrieb des Analysegeräts 1 unmittelbar nach dem Entstehen redundant auf das zusätzliche Wechsel-Speichermedium 23 geschrieben. Dies entspricht einem Normalbetrieb (Mess- oder Analysebetrieb) des ordnungsgemäß funktionierenden Analysegeräts 1.

Wenn nun eine Flash-Karte 23 in das Speicherinterface 20 des äquivalenten Analysegeräts 100 eingelegt wird, welche Flash-Karte 23 zuvor auf dem Analysegerät 1 benutzt wurde und z.B. deshalb in das neue Analysegerät 100 eingelegt wurde, weil bei dem zuvor benutzten Analysegerät 1 Probleme aufgetreten waren, so ist dieser Vorgang schematisch mit Hilfe der unterbrochenen Linie 39 in der Figur 1 dargestellt und symbolisiert die Entnahme der Flash-Karte 23 aus dem Analysegerät 1 und das Einlegen der Flash-Karte 23 in das Analysegerät 100. Anschließend werden im Analysegerät 100 die vorhin beschriebenen Start-up Prozeduren durchlaufen. Das Importieren der auf der Flash-Karte 23 vorhandenen Daten D in den internen Speicher 21 des Analysegeräts 100 ist durch die mit dem Bezugszeichen DIMP gekennzeichnete unterbrochene Linie angedeutet. Daraufhin erfolgt der Normalbetrieb des äquivalenten Analysegeräts 100.

In der weiteren Beschreibung des Verfahrens wird davon ausgegangen, dass das Analysegerät 1 bereits in der Vergangenheit erstmals in Betrieb genommen wurde. Dieser Zustand ist beispielsweise durch entsprechende Daten D in dem internen Speichermedium 21 repräsentiert.

Optional sei an dieser Stelle erwähnt, dass der Benutzer oder auch das Analysegerät 1 bzw. 100 oder auch die Flash-Karte 23 selbst ein Überschreiben der Daten D sowohl auf dem internen Speichermedium 21 als auch auf der Flash-Karte 23 verhindern kann, was jedoch in der Figur 2 nicht im Detail dargestellt ist.

Das vorstehend beschriebene Analysegerät 1 bzw. 100 und das mit ihm durchführbare Verfahren erlauben es nun, verschiedene Schadensfälle zu bewältigen. Bei einem Schadensfall I (Analysegerät 1 defekt) und/oder einem Schadensfall II (PC defekt) und/oder einem Schadensfall III (primärer Datenträger, also internes Speichermedium defekt) wird das externe Wechsel-Speichermedium 23 in einen Ersatzanalysator (z.B. äquivalentes Analysegerät 100) transferiert, installiert und dort weiterverwendet.

Wie vorstehend erörtert, kann dieses Ersatzgerät (zweiter Analysator, z.B. äquivalentes Analysegerät 100) hierbei a) ein Gerät sein, das noch nie in Betrieb genommen wurde (und somit keine solchen relevanten Daten D enthält), oder auch b) ein Gerät sein, das bereits zuvor in Betrieb genommen wurde (und somit bereits relevante Daten D basierend auf dem bisherigen Betrieb dieses Geräts aufweist), sein. Für beide Fälle sieht das erfindungsgemäße Verfahren Verfahrensschritte vor, die dem jeweiligen Fall gerecht werden.

Im Schadensfall IV (externer Datenträger, also Wechsel-Speichermedium 23 defekt) kann dieser erneuert (ausgetauscht) werden, wobei die relevanten Daten D nach wie vor auf dem internen Speichermedium 21 gespeichert sind.

Bei der Inbetriebnahme eines Analysegeräts 1 kann beispielsweise wie folgt vorgegangen werden:
a) Beim ersten Hochfahren der Analysatorsoftware wird die Existenz einer Flash- Karte 23 (z.B. als CompactFlash-Karte ausgebildet) geprüft. Ist keine CF-Karte 23 vorhanden, so kann das Analysegerät 1 nicht gestartet bzw. betrieben werden. Ist jedoch eine Flash-Karte 23 vorhanden, so wird ihre Seriennummer ausgelesen. Stimmt die Seriennummer nicht mit der in der Konfiguration abgelegten Seriennummer überein, so werden die in der Flash-Karte 23 abgelegten Daten D in die Datenbank im internen Speichermedium 21 des Analysegeräts 1 importiert. Sämtliche Änderungen werden in einem Audit-Trail protokolliert.
b) Beim ersten Hochfahren der Analysatorsoftware wird die Existenz einer Compact-Flash Karte 23 geprüft. Ist keine CF-Karte 23 vorhanden, so wird eine Warnung angezeigt, dass der Backup-Modus nicht aktiv ist und Daten D nicht redundant gespeichert werden. Der Backup-Modus kann dann beispielsweise manuell oder auch automatisch wieder aktiviert werden, sobald eine leere CF-Karte 23 im Analysegerät 1 detektiert wird.

Beim Austausch des externen Wechsel-Speichermediums 23 kann beispielsweise wie folgt vorgegangen werden:
a) Wird auf einem bereits in Betrieb genommenen Analysegerät eine Flash-Karte 23 detektiert, die eine Seriennummer aufweist, die nicht mit der in der Konfiguration gespeicherten Seriennummer übereinstimmt, so werden die relevanten Daten D, insbesondere die Konfigurations-, Benutzer- und Messdaten, aus der Datenbank das internen Speichermediums 21 automatisch auf die neue Karte 23 exportiert. Die gesamten zu sichernden Daten D werden auf die neue, funktionierende Flash-Karte 23 geschrieben.
b) Wird auf einem bereits in Betrieb genommenen Analysegerät 1 bzw. 100 eine Flash-Karte Karte 23 detektiert, die eine Seriennummer aufweist, die nicht mit der in der Konfiguration gespeicherten Seriennummer übereinstimmt, und ist diese CF-Karte 23 nicht leer, so kann der Benutzer auswählen, ob Daten D importiert oder exportiert werden sollen.

Mit Hilfe der Erfindung kann somit auch ein Verfahren zum Management und Transfer von relevanten Daten D von Analysegeräten 1 bzw. 100 beschrieben werden, wobei die Analysegeräte 1 bzw. 100 jeweils einen internen Speicher 21 besitzen und wobei sich die relevanten Daten D auf die spezifische Konfiguration eines ersten Analysegeräts 1 sowie die bislang mit diesem ersten Analysegerät 1 gemessenen Parameter-Werte und die zugehörigen Hintergrundinformationen beziehen,
umfassend die Schritte:
- Bereitstellen eines zusätzlichen externen nicht-flüchtigen Speichermediums 23 in dem ersten Analysegerät 1,
- fortlaufendes redundantes Abspeichern aller neu angelegten und/oder modifizierten relevanten Daten D auf dem externen nicht-flüchtigen Speichermedium 23 und dem internen Speicher 21 des ersten Analysegeräts 1 während des Betriebes (Analyse bzw. Messen) des ersten Analysegeräts 1,
- im Falle des Transfers der redundant gespeicherten Daten D auf ein zweites Analysegerät 100 Entnehmen des externen nicht-flüchtigen Speichermediums 23 aus dem ersten Analysegerät 1 und Einsetzen des externen nicht-flüchtigen Speichermediums 23 in das zweite Analysegerät 100,
- Einlesen der auf dem externen nicht-flüchtigen Speichermedium 23 gespeicherten relevanten Daten D, welche während des Betriebs im ersten Analysegerät 1 gespeichert wurden, in den internen Speicher 21 des zweiten Analysegeräts 100,
- Fortsetzen des Betriebs (Analyse bzw. Messen) mit dem zweiten Analysegerät 100 unter Verwendung dieser eingelesenen relevanten Daten D.

Die damit erhaltenen Vorteile der Lösung lassen sich wie folgt auflisten:
- Durch das fortlaufende redundante Speichern sämtlicher relevanter Daten D ist im Gegensatz zu bekannten Backup-Verfahren keine Logik oder zeitliche Steuerung von automatischen Synchronisationszyklen notwendig.
- Der Datenbestand kann zu jedem Zeitpunkt, gegebenenfalls zum Zeitpunkt unmittelbar vor dem Störfall rekonstruiert werden (keine Quantisierungsverluste durch manuelle oder automatische Synchronisationszyklen).
- Durch Prüfung der Existenz eines eindeutig identifizierten externen Speichermediums 23 und dessen Verwendung als Token wird verhindert, dass ein Analysegerät 1 bzw. 100 in Betrieb genommen wird, das Daten D lückenhaft oder gar nicht redundant speichern kann.
- Durch die Anforderung der Existenz eines bestimmten, eindeutigen Speichermediums 23 bei Inbetriebnahme und der implementierten Regelung, unter welchen Bedingungen ein Importszenario oder ein Exportszenario vorliegt, wird die Notwendigkeit einer manuellen Interaktion vermieden.
- Eine identische Konfiguration kann einfach auf viele Analysegeräte 1 bzw. 100 übertragen werden.
- Im Störfall kann die Aufzeichnung der oben genannten Daten D als Dokumentation und/oder Bug-Report dienen.
- Der Nutzer kann sofort auf dem zweiten Analysegerät 100 mit seiner spezifischen und gewohnten Benutzeroberfläche weitermessen, ohne manuelle Neueinstellungen auf diesem Ersatzgerät 100 durchführen zu müssen.

Auch wenn anhand der Figurenbeschreibung die Erfindung im Kontext von einem Blutanalysator erörtert wurde, kann die Erfindung auch in anderen wissenschaftlichen und/oder industriellen Einsätzen genutzt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließen die Begriffe "Stufe", "Einheit", "Modul" oder Einrichtung", etc. nicht aus, dass diese aus mehreren Komponenten bestehen, die gegebenenfalls auch räumlich verteilt sein können.

## Patentansprüche

1. Verfahren zum Verwalten von Daten von Analysegeräten, insbesondere zum Transfer von Daten zwischen einem ersten und einem zweiten Analysegerät,
wobei das erste Analysegerät Daten in einem internen Speichermedium neu anlegt oder modifiziert und während des Betriebs fortlaufend die neu angelegten oder modifizierten Daten in einem nichtflüchtigen Wechsel-Speichermedium, das direkt in einem Einschub des ersten Analysegeräts eingesetzt oder mit dem ersten Analysegerät verbunden ist, redundant speichert, und
die auf dem Wechsel-Speichermedium von dem ersten Analysegerät redundant gespeicherten Daten durch physikalischen Transfer des externen Wechsel-Speichermediums zwischen den Analysegeräten in das interne Speichermedium eines äquivalenten zweiten Analysegeräts importiert werden,
wobei die im Wechselspeichermedium redundant gespeicherten Daten Konfigurationsdaten des ersten Analysegeräts sind, insbesondere Geräteeinstellungen wie Sprache, IP-Adresse, Anzeigebildschirm, Ländereinstellungen, Energiemanagement, Netzwerkeinstellungen, berechnete und angezeigte Maßeinheiten, verwendete Messparameter, Mess-, Kalibrations- und Qualitätskontrollgrenzen, Qualitätsmessungs-Materialien, Sicherheitseinstellungen, Audioeinstellungen, geplante automatische Durchführung von Analysatorfunktionen und/oder Peripheriegeräte,
wobei bei Inbetriebnahme eines Analysegeräts die Verfügbarkeit des Wechsel-Speichermediums geprüft wird und, wenn das Wechsel-Speichermedium nicht verfügbar ist, der Betrieb nicht ermöglicht wird,
wobei bei einem verfügbaren Wechsel-Speichermedium geprüft wird, ob ein auf dem Wechsel-Speichermedium vorhandener Datenbestand aus einem anderen Analysegerät stammt und, wenn dies zutrifft, diese in dem Wechsel-Speichermedium enthaltenen redundant gespeicherten Daten in das interne Speichermedium des Analysegeräts importiert werden, und
wobei die Entscheidung, ob in dem Wechselspeichermedium redundant gespeicherte Daten in das Analysegerät importiert werden, von einer Benutzerinteraktion abhängig ist.

2. Verfahren nach Anspruch 1, wobei die Analysegeräte medizinisch-diagnostische Analysegeräte, wie beispielsweise Analysegeräte zur Analyse von Körperflüssigkeiten, insbesondere Blutanalysegeräte sind.

3. Verfahren nach Anspruch 1 oder 2, wobei die im Wechselspeichermedium redundant gespeicherten Daten weiters zumindest eine der nachfolgend angeführten Datenarten repräsentieren:
i) Messdaten, insbesondere Messresultate, Rohdaten, Bilddaten, Messsignale, und/oder weiterverarbeitete Daten,
ii) Probandendaten,
iii) Probendaten, insbesondere Proben-Identifikationskennung, Probennahmezeitpunkt, Probentyp, und/oder Abnahmetemperatur,
iv) Benutzerdaten, insbesondere Benutzer-Identifikationskennung, benutzerspezifische Berechtigungen, und/oder Benutzerprofile.

4. Verfahren nach einem der vorangehenden Ansprüche, wobei zumindest die in dem Wechsel-Speichermedium redundant gespeicherten Daten verschlüsselt sind, z.B. mittels Passwörtern und/oder Benutzernamen gegen Missbrauch gesichert sind.

5. Verfahren nach einem der vorangehenden Ansprüche, **dadurch gekennzeichnet, dass** das erste Analysegerät bei Verbindung mit einem neuen Wechsel-Speichermedium die in dem internen Speichermedium gespeicherten Daten dann in das neue Wechsel-Speichermedium exportiert, wenn dieses nicht bereits valide Daten von einem Fremd-Analysegerät enthält.

6. Verfahren nach einem der vorangehenden Ansprüche, wobei vor der ersten Inbetriebnahme eines Analysegeräts eine eindeutige Identifikationskennung des dem jeweiligen Analysegerät zugeordneten Wechsel-Speichermediums in einem internen Speichermedium des jeweiligen Analysegeräts abgespeichert wird oder eine eindeutige Identifikationskennung des Analysegeräts in einem dem jeweiligen Analysegerät zugeordneten Wechsel-Speichermediums abgespeichert wird.

7. Verfahren nach einem der vorangehenden Ansprüche, **dadurch gekennzeichnet, dass** das Wechsel-Speichermedium einen Hardware-Token umfasst, der für den Betrieb des Analysegeräts nötig ist.

8. Verfahren nach einem der vorangehenden Ansprüche, wobei das Importieren von auf dem Wechsel-Speichermedium redundant gespeicherten Daten protokolliert wird, insbesondere in einem Audit-Trail protokolliert wird.

9. Analysegerät (1, 100) zum Verwalten von Daten (D), zum Durchführen des Verfahrens gemäß einem der Ansprüche 1 bis 8, mit einem internen Speichermedium (21) zum Speichern der Daten (D), die in dem Analysegerät (1, 100) neu angelegt oder modifiziert werden, und
mit einem Speicherinterface (20) zum Zugreifen auf ein nichtflüchtiges Wechsel-Speichermedium (23), sodass mit Hilfe des Speicherinterfaces (20) während des Betriebs des Analysegeräts (1, 100) fortlaufend die neu angelegten oder modifizierten Daten (D) in dem Wechsel-Speichermedium (23) redundant speicherbar sind.

10. Analysegerät (1, 100) nach Anspruch 9, wobei das Analysegerät (1, 100) ein medizinisch-diagnostisches Analysegerät, wie beispielsweise ein Analysegerät zur Analyse von Körperflüssigkeiten, insbesondere ein Blutanalysegerät, ist.

11. Computerprogrammprodukt, das direkt in einen Arbeitsspeicher eines programmierbaren Analysegeräts (1, 100) ladbar ist und das Programmcodeabschnitte aufweist, die, wenn das Computerprogrammprodukt mit Hilfe des Analysegeräts (1, 100) ausgeführt wird, das Verfahren nach Anspruch 1 bis 8 realisieren.

12. System mit zwei Analysegeräten nach Anspruch 9 und/oder 10, wobei die in einem der beiden Analysegeräte während des Betriebs des einen Analysegeräts (1, 100) fortlaufend neu angelegten oder modifizierten Daten (D) und in dem Wechsel-Speichermedium (23) redundant gespeicherten Daten (D) zum Betrieb des anderen Analysegeräts (100, 1) dienen, wenn das andere Analysegerät (100, 1) auf das Wechsel-Speichermedium (23) zugreift.

## Claims

1. A method for managing data of analysis devices, in particular for transferring data between a first and a second analysis device,
wherein the first analysis device creates a new or modifies data in an internal storage medium and redundantly stores the newly created or modified data continuously during operation in a non-volatile removable storage medium, which is inserted directly into a bay of the first analysis device or which is connected to the first analysis device, and
wherein the data redundantly stored on the removable storage medium by the first analysis device are imported by means of physical transfer of the external removable storage medium between the analysis devices into the internal storage medium of an equivalent second analysis device,
wherein the data redundantly stored in the removable storage medium are configuration data of the first analysis device, in particular device set-up such as language, IP address, display screen, regional settings, energy management, network setting, calculated and displayed measuring units, measuring parameters used, measurement, calibration and quality control limits, quality measurement materials, security settings, audio settings, projected automated carrying out of analysator functions and/or peripheral devices,
wherein, upon start-up of an analysis device, the availability of the removable storage medium is examined, and if the removable storage medium is not available, then the operation will not be enabled,
wherein, when a removable storage medium is available, there is examined if a data set present on the removable storage medium originates from another analysis device and, if this is the case, the data contained and redundantly stored in the removable storage medium will be imported into the internal storage medium of the analysis device, and
wherein the decision whether data redundantly stored in the removable storage medium are imported into the analysis device is dependent on an interaction of the user.

2. A method according to claim 1, wherein the analysis devices are medicinal-diagnostic analysis devices such as, for example, analysis devices for analysing body fluids, in particular blood analysis devices.

3. A method according to claim 1 or 2, wherein the data redundantly stored in the removable storage medium further represent at least one of the data types listed in the following:
i) measuring data, in particular measurement results, raw data, image data, measurement signals and/or further processed data,
ii) data of a subject,
iii) sample data, in particular sample identification labelling, sample sampling time, sample type and/or sampling temperature,
iv) user data, in particular user identification labelling, user-specific authorizations and/or user profiles.

4. A method according to any of the preceding claims, wherein at least the data redundantly stored in the removable storage medium are encoded, e.g., secured against fraudulent use by means of pass words and/or user names.

5. A method according to any of the preceding claims, **characterized in that** the first analysis device, upon connection to a new removable storage medium, exports the data stored in the internal storage medium into the new removable storage medium if this does not already contain valid data of a foreign analysis device.

6. A method according to any of the preceding claims, wherein, before the first start-up of an analysis device, there is stored a distinct identification labelling of the removable storage medium assigned to the respective analysis device in an internal storage medium of the respective analysis device, or there is stored a distinct identification labelling of the analysis device in a removable storage medium assigned to the respective analysis device.

7. A method according to any of the preceding claims, **characterized in that** the removable storage medium comprises a hardware token, which is necessary for the operation of the analysis device.

8. A method according to any of the preceding claims, wherein importing of data redundantly stored on the removable storage medium is recorded, in particular recorded in an audit trail.

9. An analysis device (1, 100) for managing data (D), for carrying out the method according to any of the claims 1 to 8, having an internal storage medium (21) for storing the data (D), which are newly created or modified in the analysis device (1, 100), and having a memory interface (20) for accessing a non-volatile removable storage medium (23) such that by the aid of the memory interface (20) during the operation of the analysis device (1, 100) the newly created or modified data (D) may be continuously stored in the removable storage medium (23) in a redundant way.

10. An analysis device (1, 100) according to claim 9, wherein the analysis device (1, 100) is a medicinal-diagnostic analysis device such as, for example, an analysis device for analysing body fluids, in particular a blood analysis device.

11. A computer programme product, which may be directly loaded into a main memory of a programmable analysis device (1, 100) and which has programme code sections, which realize the method according to claims 1 to 8 if the computer programme product is carried out with the aid of the analysis device (1, 100).

12. A system having two analysis devices according to claim 9 and/or 10, wherein the data continuously newly created or modified data (D) in one of the two analysis devices during the operation of the one analysis device (1, 100) and the data (D) redundantly stored in the removable storage medium (2) serve for operating the other analysis device (100, 1) if the other analysis device (100, 1) accesses the removable storage medium (23).

## Revendications

1. Procédé pour gérer des données d'appareils d'analyse, en particulier pour transférer des données entre un premier et un deuxième appareil d'analyse,
dans lequel le premier appareil d'analyse crée nouvellement ou modifie des données dans un support de stockage interne et, pendant le fonctionnement, stocke en continu de manière redondante les données nouvellement créées ou modifiées dans un support de stockage amovible non volatil qui est inséré directement dans un tiroir du premier appareil d'analyse ou est relié au premier appareil d'analyse, et
les données stockées de manière redondante sur le support de stockage amovible par le premier appareil d'analyse sont importées dans le support de stockage interne d'un deuxième appareil d'analyse équivalent par transfert physique du support de stockage amovible externe entre les appareils d'analyse,
dans lequel les données stockées de manière redondante dans le support de stockage amovible sont des données de configuration du premier appareil d'analyse, en particulier des réglages de l'appareil tels que la langue, l'adresse IP, l'écran d'affichage, les réglages nationaux, la gestion de l'énergie, les réglages réseau, les unités de mesure calculées et affichées, les paramètres de mesure utilisés, les limites de mesure, d'étalonnage et de contrôle de qualité utilisées, les matériaux de mesure de qualité, les réglages de sécurité, les réglages audio, l'exécution automatique programmée de fonctions de l'analyseur et/ou de périphériques,
dans lequel la disponibilité du support de stockage amovible est vérifiée lors de la mise en service d'un appareil d'analyse et, si le support de stockage amovible n'est pas disponible, le fonctionnement est rendu impossible,
dans lequel, dans le cas d'un support de stockage amovible disponible, on vérifie si un ensemble de données présent sur le support de stockage amovible provient d'un autre appareil d'analyse et, si tel est le cas, ces données stockées de manière redondante contenues dans le support de stockage amovible sont importées dans le support de stockage interne de l'appareil d'analyse, et
dans lequel la décision d'importer dans l'appareil d'analyse des données stockées de manière redondante dans le support de stockage amovible dépend d'une interaction utilisateur.

2. Procédé selon la revendication 1, dans lequel les appareils d'analyse sont des appareils d'analyse pour le diagnostic médical, tels que des appareils d'analyse pour l'analyse de fluides corporels, en particulier des appareils d'analyse du sang.

3. Procédé selon la revendication 1 ou 2, dans lequel les données stockées de manière redondante dans le support de données amovible représentent en outre au moins l'un des types de données ci-après :
i) données de mesure, en particulier résultats de mesure, données brutes, données d'image, signaux de mesure et/ou données retraitées,
ii) données relatives aux sujets,
iii) données relatives aux échantillons, en particulier identificateur d'échantillons, moment de prélèvement, type d'échantillon et/ou température de prélèvement,
iv) données relatives aux utilisateurs, en particulier identificateurs d'utilisateurs, autorisations spécifiques aux utilisateurs et/ou profils d'utilisateurs.

4. Procédé selon l'une des revendications précédentes, dans lequel au moins les données stockées de manière redondante dans le support de stockage amovible sont cryptées, par exemple protégées contre une utilisation abusive par des mots de passe et/ou des noms d'utilisateur.

5. Procédé selon l'une des revendications précédentes, **caractérisé en ce que** le premier appareil d'analyse, lorsqu'il est relié à un nouveau support de stockage amovible, exporte les données stockées dans le support de stockage interne vers le nouveau support de stockage amovible si ce dernier ne contient pas déjà des données valides d'un appareil d'analyse tiers.

6. Procédé selon l'une des revendications précédentes, dans lequel, avant la première mise en service d'un appareil d'analyse, un identificateur unique du support de stockage amovible associé à l'appareil d'analyse respectif est enregistré dans un support de stockage interne de l'appareil d'analyse respectif ou un identificateur unique de l'appareil d'analyse est enregistré dans un support de stockage amovible associé à l'appareil d'analyse respectif.

7. Procédé selon l'une des revendications précédentes, **caractérisé en ce que** le support de stockage amovible comprend un jeton matériel qui est nécessaire au fonctionnement de l'appareil d'analyse.

8. Procédé selon l'une des revendications précédentes, dans lequel l'importation de données stockées de manière redondante sur le support de stockage amovible est enregistrée, en particulier dans une trace d'audit.

9. Appareil d'analyse (1, 100) pour gérer des données (D), servant à mettre en oeuvre le procédé selon l'une des revendications 1 à 8, comportant un support de stockage interne (21) pour stocker les données (D) qui sont nouvellement créées ou modifiées dans l'appareil d'analyse (1, 100), et comportant une interface mémoire (20) pour accéder à un support de stockage amovible non volatil (23) de sorte qu'à l'aide de l'interface mémoire (20), les données (D) nouvellement créées ou modifiées peuvent être stockées en continu de manière redondante dans le support de stockage amovible (23) pendant le fonctionnement de l'appareil d'analyse (1, 100).

10. Appareil d'analyse (1, 100) selon la revendication 9, lequel appareil d'analyse (1, 100) est un appareil d'analyse pour le diagnostic médical, tel qu'un appareil d'analyse pour l'analyse de fluides corporels, en particulier un appareil d'analyse du sang.

11. Produit programme d'ordinateur qui peut être chargé directement dans une mémoire de travail d'un appareil d'analyse programmable (1, 100) et qui présente des parties de code de programme qui, lorsque le produit programme d'ordinateur est exécuté à l'aide de l'appareil d'analyse (1, 100), réalisent le procédé selon les revendications 1 à 8.

12. Système comportant deux appareils d'analyse selon les revendications 9 et/ou 10, dans lequel les données (D) nouvellement créées ou modifiées en continu dans l'un des deux appareils d'analyse pendant le fonctionnement d'un appareil d'analyse (1, 100) et les données (D) stockées de manière redondante dans le support de stockage amovible (23) servent au fonctionnement de l'autre appareil d'analyse (100, 1) lorsque l'autre appareil d'analyse (100, 1) accède au support de stockage amovible (23).
